# EUROPÄISCHE PATENTANMELDUNG

(11) **EP 0 896 980 A1**
(43) Veröffentlichungstag der Anmeldung: **17.02.1999**
(21) Anmeldenummer: 98114590.7
(22) Anmeldetag: 04.08.1998
(51) Int. Cl.: C08K 5/3435, C07C 39/15, C08K 5/353, C07D 211/00, C07D 498/10

(54) **Neue Lichtschutzmittel auf Basis von Calix[n]arenen und Resorc[n]arenen**

(30) Priorität: 13.08.1997 DE 19734964
(71) Anmelder: Clariant GmbH, 65929 Frankfurt am Main (DE)
(72) Erfinder: Mehrer, Mathias, Dr., 86456 Gablingen (DE)

(57) **Zusammenfassung**

Die vorliegende Erfindung bezieht sich auf neue Stabilisatoren der allgemeinen Formeln (I) und (II) 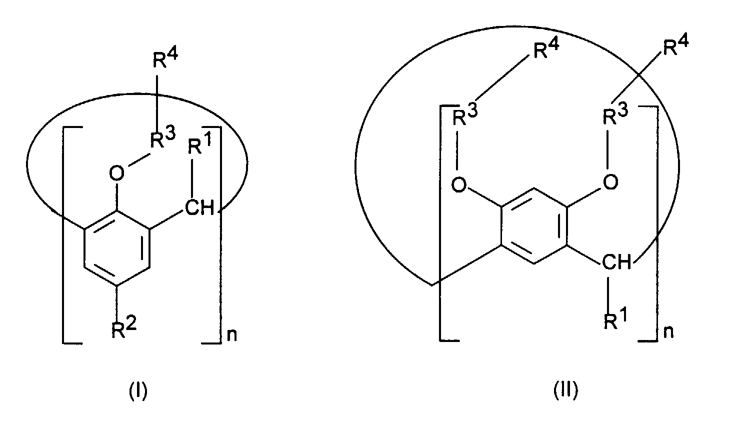 wobei die Substituenten die in der Beschreibung definierte Bedeutung haben. Die neuen Verbindungen sind hochwirksame Lichtschutzmittel für organisches Material.

## Beschreibung

Die Stabilisierung von polymerem Material gegen den zerstörerischen Einfluß energiereicher Strahlung ist Gegenstand intensiver Forschungen. In den letzten Jahren wurde eine Vielzahl von Lichtschutzmitteln auf Basis sterisch gehinderter Amine (HALS = 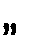 **h**indered **a**mine **l**ight **s**tabilizer") entwickelt, die die Stabilisierung von polymerem Material in ausgezeichneter Art und Weise gewährleisten. Dennoch leiden gerade niedermolekulare Stabilisatoren dieser Substanzklasse unter dem Nachteil der leichten Ertrahierbarkeit sowie hoher Flüchtigkeit aus dem zu stabilisierenden Material, so daß nach einiger Anwendungszeit unerwünschte vorzeitige Zersetzung des Materials eintritt. Aus diesem Grund wurde eine beträchtliche Anzahl polymerer HALS-Stabilisatoren hergestellt, die die dargestellten Nachteile nicht oder nur in geringerem Maße besitzen. Als exemplarische Beispiele seien Stabilisatoren erwähnt, wie sie z. B. in EP-A-93693, DE-A-2 719 131, EP-A-343 717 und EP-A-402 889 beschrieben sind. Es besteht jedoch ein steter Bedarf an neuen leistungsfähigen Stabilisatoren, welche über verbesserte Lichtschutz- oder zusätzliche bessere Gebrauchseigenschaften verfügen.

In der neueren chemischen Literatur wird die facettenreiche Chemie der Calix[n]arene und Resorc[n]arene, d. h. von cyclischen Kondensationsprodukten substituierter Phenole oder Resorcine mit aliphatischen oder aromatischen Aldehyden beschrieben. Als hervorragende exemplarische Übersichtsartikel, welche sich ausführlich mit der Chemie dieser Substanzklasse beschäftigen, sei auf die Literaturstellen Chemie in unserer Zeit, 25. Jahrgang (1991); Nr. 4; Seite 195 und Angew. Chem. 1995, 107, 785-818 verwiesen. Maßgeschneiderte, chemisch modifizierte Calix[n]arene oder Resorc[n]arene eignen sich potentiell als hochspezifische Liganden für die Analytik, die medizinische Diagnostik oder auch zur Dekontaminierung industrieller Abwässer. Ein weiteres interessantes Anwendungsgebiet ist die Herstellung neuer organischer Materialien mit außergewöhnlichen Eigenschaften (nichtlineare optische Eigenschaften, Entwicklung neuartiger Katalysatortypen usw.). In der Patentliteratur werden Calix[n]arene z. B. in Kombination mit P(III)-Reagentien als hervorragende Verarbeitungsstabilisatoren für polymere Materialien beschrieben. Ein weiterer industrieller Anwendungsbereich besteht in der Verwendung eben solcher Phosphite als Liganden für Rhodium- oder Ruthenium-Katalysatoren bei der Hydroformylierungsreaktion, einer großtechnisch bedeutenden Reaktion (WO 95/00525 und DE-A-4 321 194).

Überraschenderweise konnte festgestellt werden, daß sich neue, hochwirksame Lichtschutzmittel auf Basis von Calix[n]arenen oder Resorc[n]arenen herstellen lassen.

Gegenstand der Erfindung sind deshalb Verbindungen der allgemeinen Strukturformeln (I) und (II), 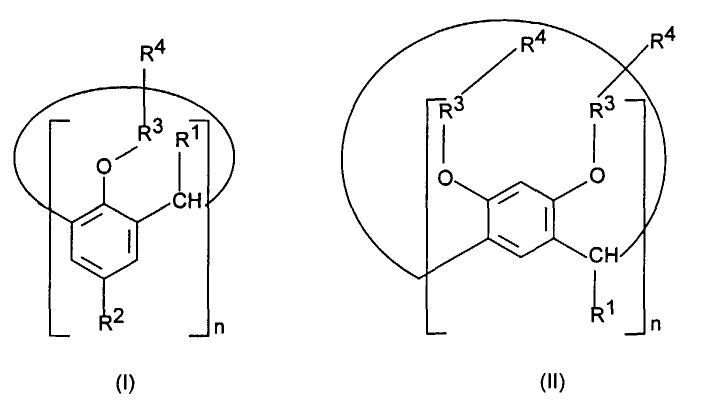 wobei
- n: eine Zahl von 4 bis 8, im Falle der Formel (I) vorzugsweise 4, 6, 8 und im Falle der Formel (II) vorzugsweise 4 bedeutet,
- R¹: Wasserstoff, C₁-C₁₈-, vorzugsweise C₁-C₁₀-, insbesondere C₁-C₄-Alkyl, C₃-C₁₀-, vorzugsweise C₅-C₆-Cycloalkyl oder einen ein- oder mehrfach durch Wasserstoff, C₁-C₄-Alkyl, Trifluormethyl, C₁-C₄-Alkoxy, Halogen, Cyan, Carboxy, Nitro, Amino, C 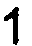 -C 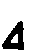 -Alkylamino, C 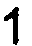 -C 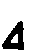 -Dialkylamino, Acyl oder Acyloxy substituierten aromatischen Rest bedeutet,
- R²: lineares oder verzweigtes C₁-C₁₈-Alkyl, vorzugsweise C₁-C₆-Alkyl, insbesondere aber den tert. Butylrest oder den iso-Amylrest bedeutet,
- R³: ein Rest -CH₂-C(O)-, -C(O)-, -C(O)-C(O)-, -CH₂-CHR¹- oder -(CH₂- CH₂-O)ₓ-(mit x = 1-8), vorzugsweise -CH₂-C(O)- oder -C(O)- und insbesondere -CH₂-C(O)- ist,
- R⁴: einen Rest der allgemeinen Strukturformeln (III) bis (VII) 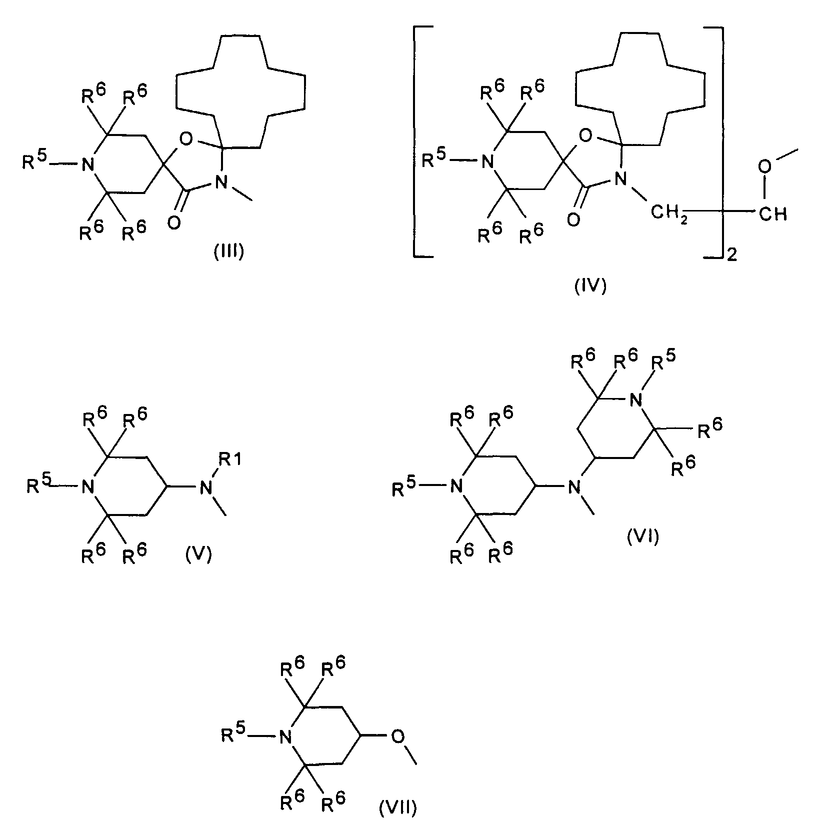 vorzugsweise der Formeln (V) oder (VII) bedeutet, wobei
- R¹: die oben angegebene Bedeutung hat,
- R⁵: Wasserstoff, eine C₁-C₂₀-, vorzugsweise eine C₁-C₅-Alkylgruppe, insbesondere eine Methylgruppe, ein Sauerstoffradikal O*, -OH, -NO, -CH₂CN, Benzyl, Allyl, eine C₁-C₃₀-, vorzugsweise eine C₁-C₁₀-Alkyloxygruppe, eine C₅-C₁₂-, vorzugsweise eine C₅-C₆-Cycloalkyloxygruppe, eine C₃-C₁₀-, vorzugsweise eine C₃-C₆-Alkenylgruppe, eine C₃-C₆-Alkinylgruppe, eine C₁-C₁₀-, vorzugsweise eine C₁-C₅-Acylgruppe, Halogen, ein unsubstituierter oder durch C₁-C₄-, vorzugsweise durch C₁-C₂-Alkyl substituierter Phenylrest ist,
- R⁶: Wasserstoff oder eine C₁-C₁₂-Alkylgruppe, vorzugsweise eine C₁-C₄-Alkylgruppe, insbesondere eine Methylgruppe ist.

Die Synthese dieser Lichtschutzmittel gelingt, indem die zugrundeliegenden, leicht herstellbaren Calix[n]arene oder Resorc[n]arene an den phenolischen Sauerstoffunktionen vollständig mit Spacergruppen versehen werden, welche als 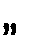 Ankerplattform" die Reaktion mit Molekülen auf Basis von 2,2,6,6-Tetraalkylpiperidinen ermöglicht. Die Einheiten der 2,2,6,6-Tetraalkylpiperidine verleihen den erfindungsgemäßen Verbindungen eine hervorragende Lichtschutzwirkung. Die erfindungsgemäße neue Verbindungsklasse zeichnet sich durch eine geringe Flüchtigkeit aus den zu stabilisierenden Materialien bei gleichzeitiger geringer Extrahierbarkeit im Kontakt mit extrahierenden Medien aus.

Die Erfindung bezieht sich auch auf das allgemeine Herstellungsverfahren von Verbindungen der Formel (I) und (II) sowie deren Verwendung zur Stabilisierung von organischem Material. Unter organischem Material sind beispielsweise Polymere, Anstrichmittel, Öle und Lacke zu verstehen, insbesondere Polymere.

Die Herstellung von Verbindungen der Formel (I) bzw. (II) gelingt, indem Calix[n]arene der allgemeinen Formel (VIII) oder Resorc[n]arene der allgemeinen Formel (IX), welche auf literaturbekannten Wegen zugänglich sind, 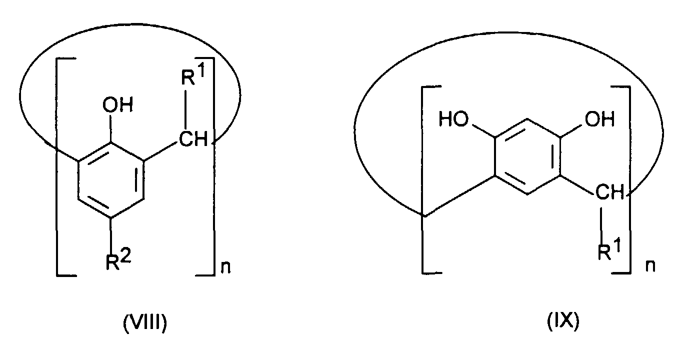 durch Umsetzung mit n oder mehr Äquivalenten eines geeigneten Reagenzes R in ein reaktives Molekül der allgemeinen Formeln (X) bzw. (XI) 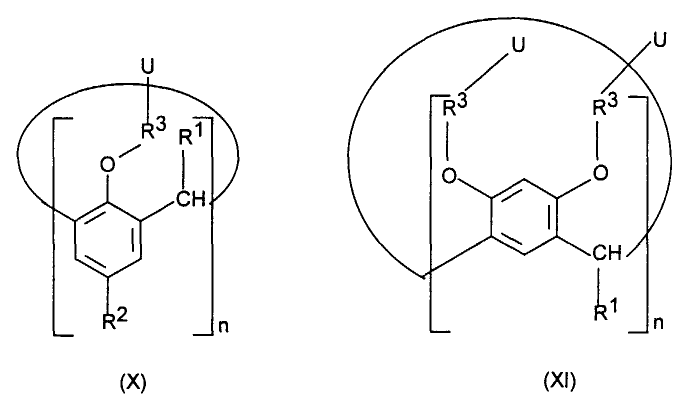 übergeführt werden,
wobei das Reagenz R z. B. Hal-CH₂-C(O)-U, U-C(O)-U, U-C(O)-C(O)-U, Ethylenoxid oder Hal-CH₂-CHR¹-Hal wobei R¹ die obige Bedeutung hat und das Symbol U Halogen, OR¹ oder N(R¹)₂ mit R¹ mit obiger Definition, bedeutet.
Bei dem Reagenz R handelt es sich z. B. um Phosgen, Bromessigsäurealkylester, Chloressigsäurealkylester, Chlorameisensäuremethylester, Oxalsäure, Oxalsäurehalogenidmonoester oder Ethylenoxid.

Die reaktiven Zwischenstufen der allgemeinen Formeln (X) und (XI) können dann in einem weiteren Schritt mit Verbindungen R⁴-H, d.h. den Formeln (III') bis (VII') 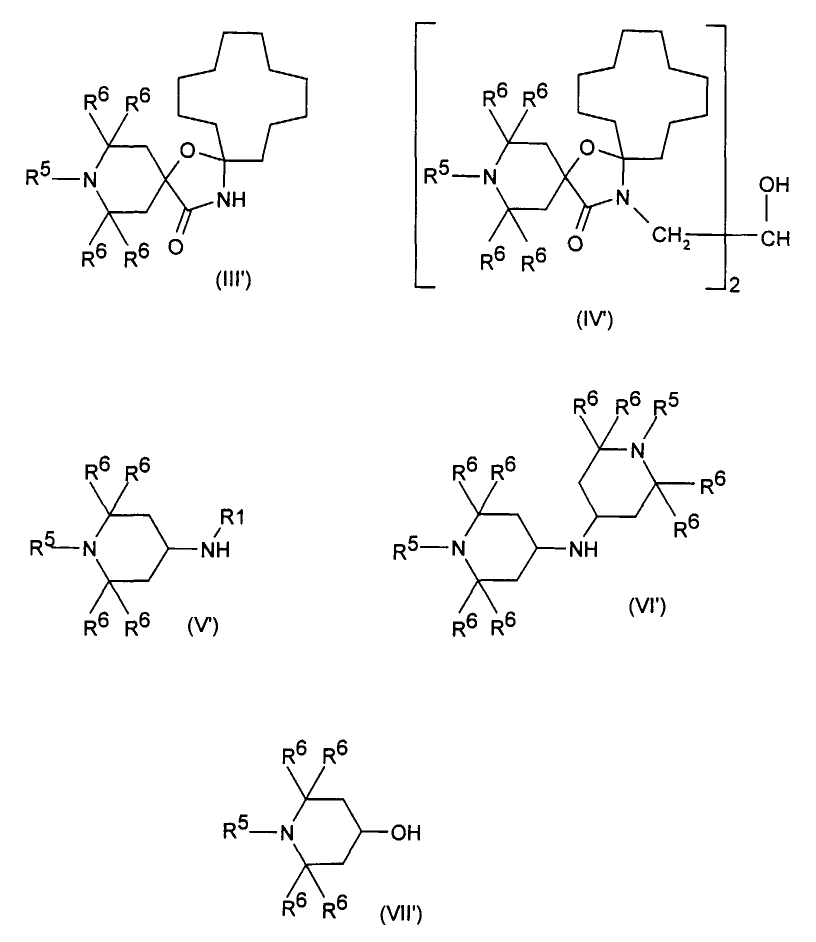 oder auch deren in situ hergestellten Anionen, welche vorzugsweise aus R⁴-H mittels einer geeigneten Base (Natronlauge, Kalilauge, Natriumhydrid oder Kalium-tert.-butylalkoholat) hergestellt werden können, umgesetzt werden.
Die besagten Umsetzungen erfolgen in protischen oder aprotischen, vorzugsweise aprotischen organischen, vorzugsweise hochsiedenden Lösemitteln, Kohlenwasserstoffen, insbesondere Toluol, Xylol oder auch Gemischen hieraus oder cyclischen Ethern wie z. B. THF oder Dioxan oder deren Gemischen mit Kohlenwasserstoffen. Die Reaktionstemperatur liegt zwischen 0°C und der Siedetemperatur der eingesetzten Lösemittel. Die Aufarbeitung erfolgt nach gängigen Methoden der Isolierung organischer Substanzen.

Die erfindungsgemäßen Verbindungen der allgemeinen Formeln (I) und (II) eignen sich in hervorragender Weise zum Stabilisieren von organischem Material gegen die Einwirkung von Licht, Sauerstoff und Wärme. Die erfindungsgemäßen Verbindungen eignen sich, aufgrund ihrer hevorragenden komplexierenden Eigenschaften zusätzlich zur Desaktivierung von Metallresten in dem zu stabilisierenden Material. Bei solchen Metallrückständen handelt es sich z. B. um Katalysatorreste (Al, Cr, V, Fe, Ti), welche nach der Polymerisation heute oft im Kunststoff verbleiben und den photochemischen Abbau des Polymers enorm beschleunigen. Die erfindungsgemäßen Verbindungen der allgemeinen Formeln (I) und (II) können dem zu stabilisierenden polymeren Material vor, während oder nach der Polymerisation in fester, geschmolzener, in Lösungsmitteln gelöster Form oder auch als Masterbatch zugegeben werden. Die Lösungen können den neuen Stabilisator z. B. in 10 - 50 %iger Konzentration enthalten; ein Masterbatch eignet sich besonders gut, wenn es den neuen Stabilisator in einer Konzentration von 1 bis 80 %, vorzugsweise aber von 5 - 30 % enthält, der Rest im Masterbatch ist ein mit dem zu stabilisierenden Polymer verträgliches Polymer. Sowohl die Lösung, als auch das Masterbatch kann zusätzlich noch weitere Stabilisatoren oder Effektstoffe, z. B. Pigmente, Säurefänger oder Füllstoffe, enthalten. Die neuen Stabilisatoren werden vorzugsweise so eingesetzt, daß sie im zu stabilisierenden Polymer in einer Konzentration von 0,001 bis 5 Gew.-%, vorzugsweise von 0,02 bis 1 Gew.-%, bezogen auf das organische Material, entweder alleine oder in Kombination mit weiteren Additiven enthalten sind. Unter organischem Material sind beispielsweise Vorprodukte für Kunststoffe, Anstrichmittel, Lacke und Öle, insbesondere jedoch Kunststoffe, Anstrichmittel, Lacke und Öle selbst zu verstehen.

Im besonderen eignen sich die Stabilisatoren der allgemeinen Formel (I) oder (II) zum Stabilisieren von Folien, Fasern, Bändchen, Multifilamenten, Geweben, Spritzgußartikeln, Pulverlacken, Druckfarben, Tonerfarben, photographischem Material, Pigmenten, Holzbeizen, Leder, Anstrichfarben für Gebäude, Schutzanstrichen für Stahlkonstruktionen, Schmierölen, Maschinenölen, Bitumen oder Asphalt.

Gegenstand der vorliegenden Erfindung sind außerdem gegen die Einwirkung von Licht, Sauerstoff und Wärme stabilisierte organische Materialien, insbesondere Kunststoffe, Anstrichmittel, Lacke und Öle, welche Verbindungen der allgemeinen Formeln (I) oder (II) in den oben angegebenen Konzentrationen enthalten.

Beispiele für derartige Materialien sind in der deutschen Patentanmeldung Nr. 19 719 944.5 auf den Seiten 44 bis 50 beschrieben, worauf hier ausdrücklich Bezug genommen wird.

Das durch die erfindungsgemäßen Verbindungen der allgemeinen Formel (I) oder (II) stabilisierte organische Material kann gegebenenfalls noch weitere Additive enthalten, beispielsweise Antioxidantien, Lichtstabilisierungsmittel, Metalldesaktivatoren, antistatische Mittel, flammhemmende Mittel, Pigmente und Füllstoffe. Antioxidantien und Lichtstabilisatoren, die neben den erfindungsgemäßen Verbindungen oder Kombinationen zugesetzt werden, sind beispielsweise Verbindungen auf der Basis sterisch gehinderter Amine oder sterisch gehinderter Phenole oder Schwefel oder Phosphor enthaltende Costabilisatoren. Als geignete zusätzliche in Kombination einsetzbare Additive sind beispielsweise Verbindungen verwendbar, wie sie in der deutschen Patentanmeldung Nr. 19 719 944.5 auf den Seiten 51 bis 65 beschrieben sind, worauf hier ausdrücklich Bezug genommen wird.

Die Additive der allgemeinen Formel (I) und (II) werden nach den allgemein üblichen Methoden in die organischen Polymere eingearbeitet. Die Einarbeitung kann beispielsweise durch Einmischen oder Aufbringen der Verbindungen und gegebenenfalls weiterer Additive in oder auf das Polymere unmittelbar nach der Polymerisation oder in die Schmelze vor oder während der Formgebung erfolgen. Auch durch Aufbringen der gelösten oder dispergierten Verbindungen auf das Polymere direkt oder Einmischen in eine Lösung, Suspension oder Emulsion des Polymeren, gegebenenfalls unter nachträglicher Verdunstung des Lösemittels, kann die Einarbeitung erfolgen. Die Verbindungen sind auch wirksam, wenn sie in ein bereits granuliertes Polymer nachträglich in einem gesonderten Verarbeitungsschritt eingebracht werden.

### Beispiele:

Die nachfolgenden Beispiele sollen die Erfindung näher erläutern, ohne diese in irgendeiner Weise näher einzuschränken. Alle hergestellten Edukte der Formel (X) und (XI) konnten anhand ihrer ¹H- und ¹³C-NMR-Spektren eindeutig charakterisiert werden. Die erfindungsgemäßen Produkte der Formeln (I') und (II') wurden ebenfalls anhand ihrer ¹H-NMR und ¹³C-NMR-Spektren eindeutig charakterisiert. Die Daten der angefertigten C,H,N,O Elementaranalysen belegen zusätzlich die Richtigkeit der angegebenen Strukturen.

### Verbindungen der Formel (X'):

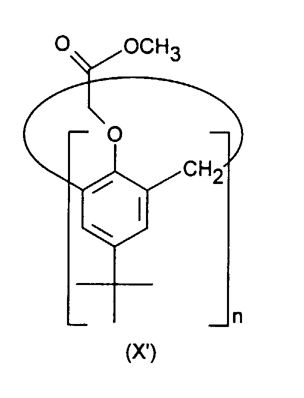

### Beispiel 1: (n=4)

19. 5 g (0.03 mol) p-tert-Butylcalix[4]aren werden in einem Gemisch aus 150 ml wasserfreiem Xylol und 100 ml wasserfreiem THF vorgelegt und bei Raumtemperatur werden 4.0 g (0.132 mol) Natriumhydrid (80 %ig) portionsweise in fester Form zugegeben. Nach Abklingen der exothermen Reaktion und der Wasserstoffentwicklung wird THF abdestilliert und der Ansatz erneut auf Raumtemperatur (RT) abgekühlt. Nach Zugabe von 36,7 g (0,24 mol) Bromessigsäuremethylester wird der Ansatz weitere 5 h auf 80°C gehalten, abgekühlt und vom Lösemittel Xylol befreit. Der Rückstand wird in 100 ml Methylenchlorid aufgenommen, zweimal mit je 100 ml Wasser extrahiert, die organische Phase über Natriumsulfat getrocknet, filtriert und vom Lösemittel und von Bromessigsäuremethylester befreit. Der Rückstand wird aus 100 ml n-Butanol umkristallisiert und liefert 17,4 g (61.9%) eines beigen, kristallinen Rückstandes vom Schmelzpunkt 207-210°C.

### Beispiel 2: n = 6

Analog Beispiel 1 aus 29,2 g (0,03 mol) p-tert-Butylcalix[6]aren, 6,0 g (0,20 mol) Natriumhydrid (80 %ig) und 55,1 g (0,36 mol) Bromessigsäuremethylester in 250 ml wasserfreiem THF. Kristallisation des Rückstandes aus 200 ml Ethanol liefert 22,6 g (53,6%) beige Kristalle vom Schmelzpunkt 203-206°C.

### Beispiel 3: n = 8

Analog Beispiel 1 aus 38,9 g (0,03 mol) p-tert-Butylcalix[8]aren, 8,1 g (0,26 mol) Natriumhydrid (80 %ig) und 73,4 g (0,48 mol) Bromessigsäuremethylester in 250 ml wasserfreiem THF. Kristallisation des Rückstandes aus 200 ml Isopropanol ergibt 35,7 g (53,6%) farblose Kristalle vom Schmelzpunkt 198-202°C.

### Verbindungen der Formeln (I')

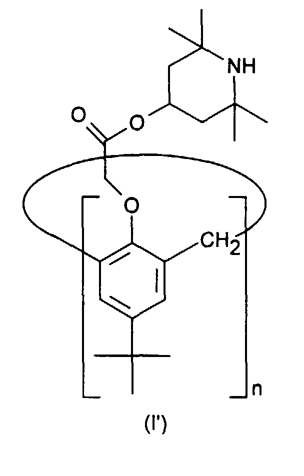

### Beispiel 4: n = 4

7, 0 g (7,5 mmol) der Verbindung aus Beispiel 1 und 4,7 g (30 mmol) 2,2,6,6-Tetramethyl-piperidin-4-ol werden in 100 ml wasserfreiem Xylol bei RT vorgelegt und 0,5 g (2 mmol) Dibutylzinnoxid zugegeben. Die Reaktionsmischung wird zum Sieden erhitzt, wobei das bei der Reaktion entstehende Methanol über eine Vigreux-Kolonne abdestilliert wird. Nach 14 h Reaktionszeit wird der Ansatz durch Destillation vom Lösemittel befreit und der Rückstand aus Aceton umkristallisiert. Der Schmelzpunk: der beigen Mikrokristalle (4,6 g; 42,7%) beträgt 127-132°C.

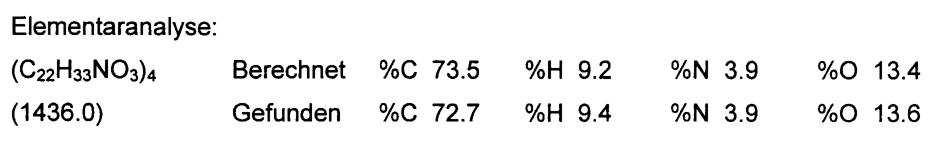

### Beispiel 5: n = 6

Analog Beispiel 4 aus 14,0 g (0,01 mol) der Verbindung aus Beispiel 2, 9,5 g (0,06 mol) 2,2,6,6-Tetramethylpiperidin-4-ol und 0,5 g (2 mmol) Dibutylzinnoxid in 100 ml wasserfreiem Xylol. Der Rückstand wird nach Abdestillation des Lösemittels heiß in 100 ml Heptan gelöst und nach dem Abkühlen filtriert. Das Filtrat wird vom Lösemittel befreit und der Rückstand pulverisiert. Der Schmelzpunkt des beigen, spröden Harzes (13,7 g; 63,9 %) beträgt 121-125°C.

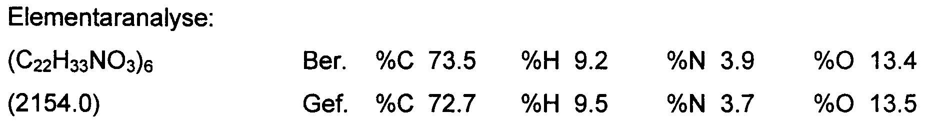

### Beispiel 6: n = 8

Analog Beispiel 4 aus 18,7 g (0,01 mol) der Verbindung aus Beispiel 3, 12,6 g (0,08 mol) 2,2,6,6-Tetramethylpiperidin-4-ol und 0,5 g (2 mmol) Dibutylzinnoxid in 150 ml wasserfreiem Xylol. Nach Abdestillation des Lösemittels wird der Rückstand heiß in 200 ml Heptan gelöst und nach dem Abkühlen von ungelösten Anteilen filtriert. Das Filtrat wird vom Lösemittel befreit und der Rückstand getrocknet und pulverisiert. Der Schmelzpunkt der beigen, klaren Harzes (25.9 g; 90,1 %) beträgt 118-133°C.

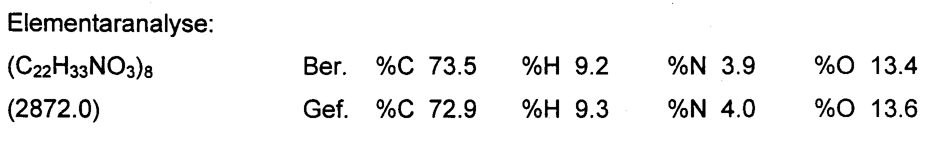

### Verbindungen der Formeln (I'')

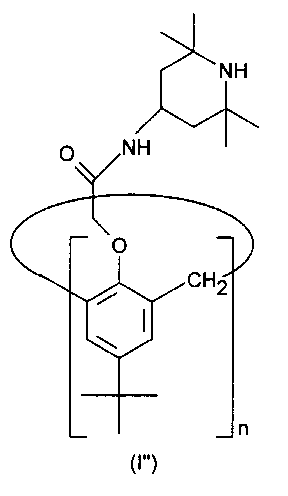

### Beispiel 7: n = 4

9, 4 g (0,01 mol) der Verbindung aus Beispiel 1 und 6,3 g (0,04 mol) 4-Amino-2,2,6,6-tetramethylpiperidin werden in 100 ml wasserfreiem Xylol bei RT vorgelegt und 0,5 g (2 mmol) Dibutylzinnoxid zugegeben. Die Reaktionsmischung wird zum Sieden erhitzt, wobei das bei der Reaktion entstehende Methanol über eine Vigreux-Kolonne kontinuierlich abdestilliert wird. Nach 12 h Reaktionszeit wird der Ansatz durch Abdestillieren vom Lösemittel befreit und der Rückstand mit heißem Heptan verrührt und filtriert. Das Filtrat wird eingeengt und der Rückstand getrocknet und pulverisiert. Der Schmelzbereich des beigen Harzes (13,9 g; 97,0%) beträgt 215-221°C.

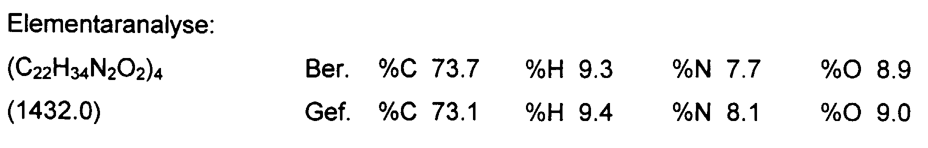

### Beispiel 8: n = 6

Analog Beispiel 7 aus 14,0 g (0,01 mol) der Verbindung aus Beispiel 2, 9,4 g (0,06 mol) 4-Amino-2,2,6,6-tetramethylpiperidin und 0,5 g (2 mmol) Dibutylzinnoxid in 100 ml wasserfreiem Xylol. Der Schmelzbereich des beigen Harzes (21,5 g; 100%) beträgt 141-161°C.

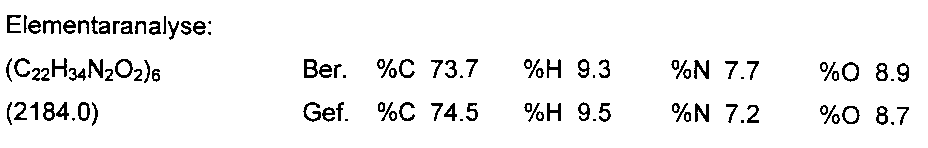

### Beispiel 9: n = 8

Analog Beispiel 7 aus 9,4 g (5 mmol) der Verbindung aus Beispiel 3, 6,3 g (0,04 mol) 4-Amino-2,2,6,6-tetramethylpiperidin und 0,5 g (2 mmol) Dibutylzinnoxid in 100 ml wasserfreiem Xylol. Nach Entfernung des Lösemittels wird der verbleibende Rückstand aus Acetonitril umkristallisiert. Der Schmelzpunkt des beigen Feststoffs (12,5 g; 87,0%) beträgt 216-249°C.

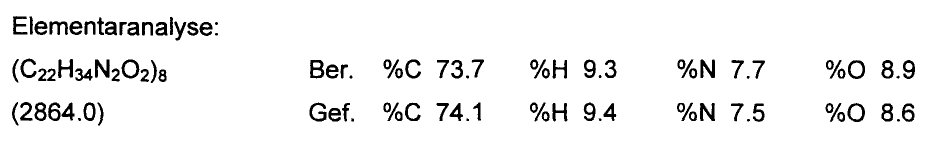

### Verbindungen der Formeln (I''')

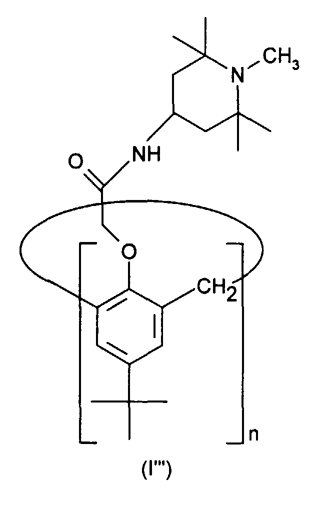

### Beispiel 10: n = 4

7,0 g (7,5 mmol) der Verbindung aus Beispiel 1 und 5,1 g (0,03 mol) 1,2,2,6,6-Pentamethylpiperidin-4-ol und 0,5 g (2 mmol) Dibutylzinnoxid werden bei RT in 100 ml wasserfreiem Xylol vorgelegt. Die Reaktionsmischung wird zum Sieden erhitzt, wobei das bei der Reaktion entstehende Methanol über eine Vigreux-Kolonne abdestilliert. Nach 16 h Reaktionszeit wird der Ansatz zur Trockene eingeengt und der Rückstand aus 50 ml Aceton umkristallisiert. Der Schmelzbereich des weißen Feststoffs (5,0 g; 44,7%) beträgt 125-130°C.

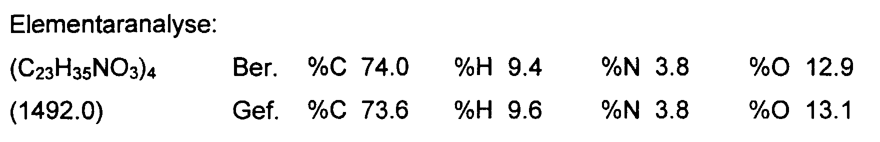

### Beispiel 11: n = 6

Analog Beispiel 10 aus 14,1 g (0,01 mol) der Verbindung aus Beispiel 2, 10,3 g (0,06 mol) Pentamethylpiperidin-4-ol und 0,5 g (2 mmol) Dibutylzinnoxid in 150 ml wasserfreiem Xylol. Nach Abdestillieren des Lösemittels wird der Rückstand aus 200 ml DMF umkristallisiert. Der Schmelzpunkt des weißen Feststoffs (12,1 g; 54,0%) beträgt 272-275°C.

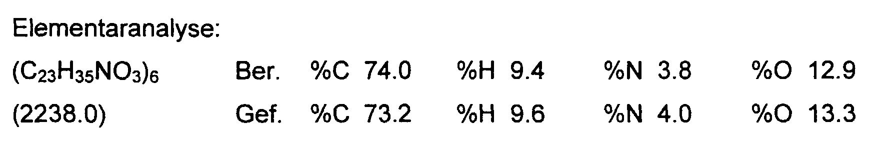

### Beispiel 12: n = 8

Analog Beispiel 10 aus 11,2 g (6 mmol) der Verbindung aus Beispiel 3, 8,2 g (0,05 mol) Pentamethylpiperidin-4-ol und 0,5 g (2 mmol) Dibutylzinnoxid in 150 ml wasserfreiem Xylol. Nach Abdestillieren des Lösemittels wird der Rückstand aus Methanol umkristallisiert. Der Schmelzbereich des beigen Pulvers (16,4 g; 91,5 %) beträgt 121-164°C.

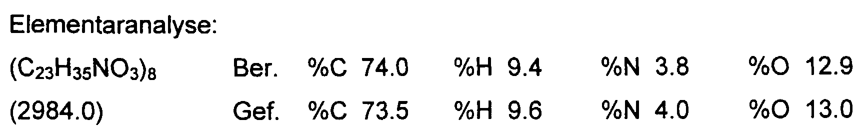

### Beispiel 13: Lichtstabilisierende Wirkung in Polypropylenfolien:

100 Gewichtsteile unstabilisiertes Polypropylen ®Hostalen PPK werden zusammen mit 0,2 Gewichtsteilen Calciumstearat, 0,1 Gewichtsteilen Tris-(2,4-di-tert-butylphenyl)-phosphit (®Hostanox PAR 24) und 0,2 Gewichtsteilen des zu prüfenden Stabilisators in einem Brabender-Mischer 10 min bei 200°C und 20 U/min geknetet. Aus dieser Mischung wurde bei 190°C eine 100 µm starke Preßfolie hergestellt und die auf diese Weise erhaltenen Prüfkörper in einem Bewitterungsgerät (®Xenotest 1200) belichtet. Als getestetes Kriterium der Stabilität der Folie wurde die Zeit bis zur Zunahme des Carbonylindexes um 1.0 Einheiten herangezogen. Die Zunahme des Carbonylindexes mit der Zeit ist hierbei ein Maß für die Abnahme der mechanischen Eigenschaften der betreffenden Folie. Der Carbonylindex CO wurde gemäß der Formel CO = E 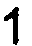 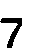 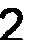 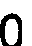 /E 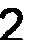 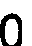 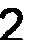 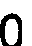 bestimmt. Zu Vergleichszwecken wurde eine Folie unter den gleichen Bedingungen, jedoch ohne den Zusatz der erfindungsgemäßen Stabilisatoren getestet. Die Versuchsergebnisse sind in Tab. 1 zusammengestellt:

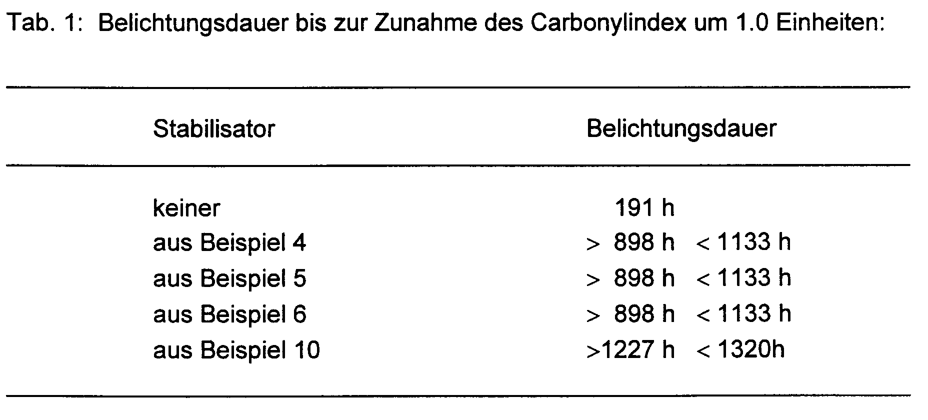

Die Ergebnisse der künstlichen Bewitterung unterstreichen die sehr gute Lichtschutzwirkung der erfindungsgemäßen Stabilisatoren.

## Patentansprüche

1. Neue Stabilisatoren der allgemeinen Formeln (I) und (II) 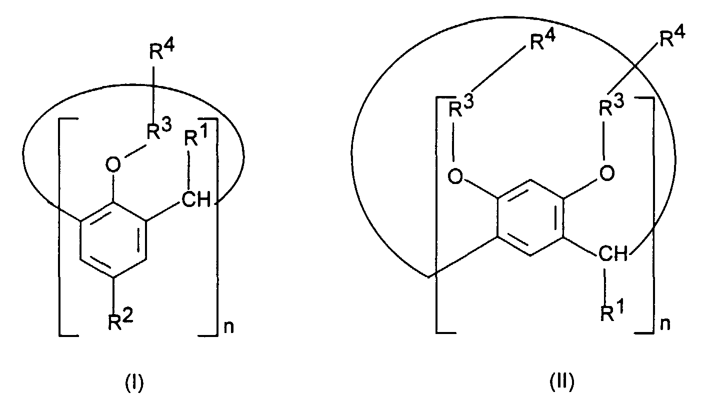 wobei
n eine Zahl von 1 bis 8 bedeutet,
R¹ Wasserstoff, C₁-C₁₈-Alkyl, C₃-C₁₀-Cycloalkyl oder einen ein- oder mehrfach durch Wasserstoff, C₁-C₄-Alkyl, Trifluormethyl, C₁-C₄-Alkoxy, Halogen, Cyan, Carboxy, Nitro, Amino, C 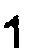 -C 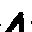 Alkylamino, C 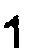 -C 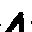 -Dialkylamino, Acyl oder Acyloxy substituierten aromatischen Rest bedeutet,
R² lineares oder verzweigtes C₁-C₁₈-Alkyl bedeutet,
R³ ein Rest -CH₂-C(O)-, -C(O)-, -C(O)-C(O)-, -CH₂-CHR¹- oder -(CH₂- CH₂-O)ₓ- mit x = 1 - 8 ist,
R⁴ einen Rest der allgemeinen Strukturformeln (III) bis (VII) bedeutet 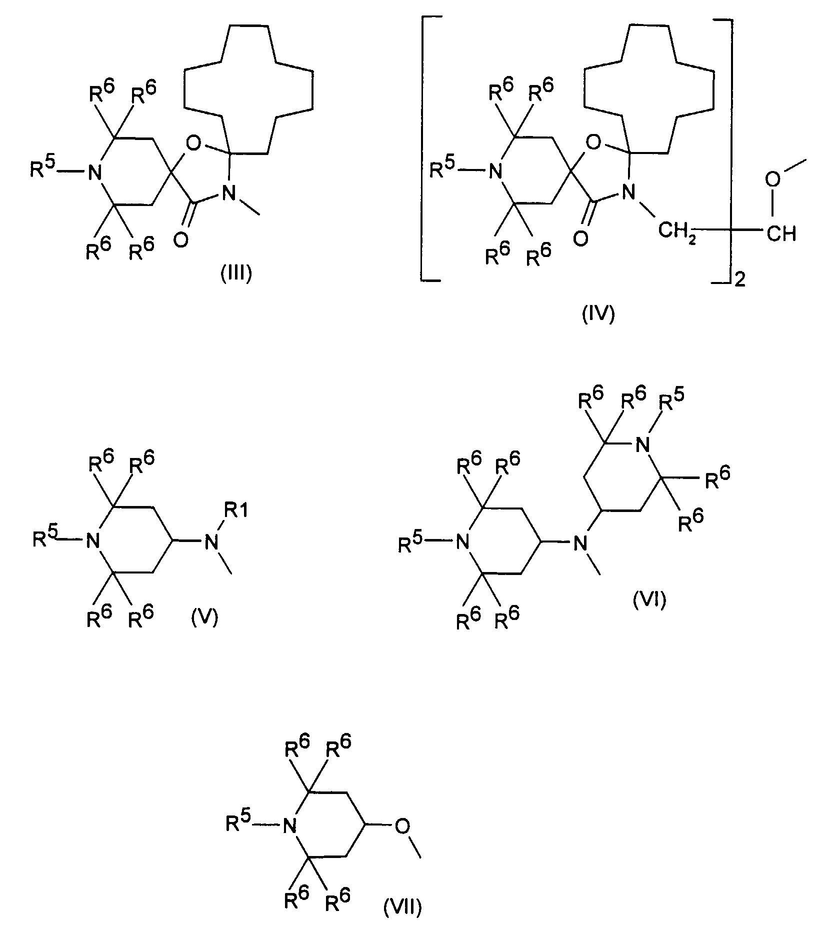 wobei
R¹ die obige Bedeutung hat,
R⁵ Wasserstoff, eine C₁-C₂₀-Alkylgruppe, ein Sauerstoffradikal O*, -OH, -NO, -CH₂CN, Benzyl, Allyl, eine C₁-C₃₀ -Alkyloxygruppe, eine C₅-C₁₂-Cycloalkyloxygruppe, eine C₃-C₁₀ -Alkenylgruppe, eine C₃-C₆-Alkinylgruppe, eine C₁-C₁₀- Acylgruppe, Halogen, ein unsubstituierter oder durch C₁-C₄-Alkyl substituierter Phenylrest ist,
R⁶ Wasserstoff oder eine C₁-C₁₂-Alkylgruppe ist.

2. Stabilisatoren gemäß Anspruch 1, dadurch gekennzeichnet, daß
R¹ Wasserstoff, C₁-C₁₀-Alkyl, C₅-C₆-Cycloalkyl,
R² lineares oder verzweigtes C₁-C₆-Alkyl,
R³ -CH₂-C(O)-, -C(O)-, -(CO)-(CO)-,
R⁴ einen Rest der allgemeinen Strukturformeln (III) bis (VII) bedeutet, wobei
R⁵ Wasserstoff, C₁-C₅-Alkyl, C₁-C₅-Alkyloxy, C₅-C₆-Cycloalkyloxy,
R⁶ C₁-C₄-Alkyl und
n 4, 6, 8 bedeutet

3. Stabilisatoren gemäß Anspruch 1, dadurch gekennzeichnet, daß
R¹ Wasserstoff, C₁-C₄-Alkyl,
R² t-Butyl,
R³ -CH₂-C(O)-,
R⁴ einen Rest der allgemeinen Strukturformel (III) bis (VII) bedeutet, wobei
R⁵ Wasserstoff, Methyl,
R⁶ Methyl,
n 4, 6, 8 in Formel (I) und 4 in Formel (II) bedeutet.

4. Verfahren zur Herstellung der Verbindungen der allgemeinen Strukturformel (I) und (II), dadurch gekennzeichnet, daß
Calix[n]arene der allgemeinen Formel (VIII) oder Resorc[n]arene der allgemeinen Formel (IX), 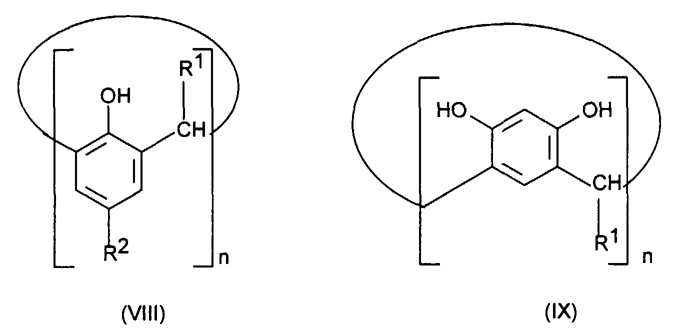 durch Umsetzung mit n oder mehr Äquivalenten eines geeigneten Reagenzes R in ein reaktives Molekül der allgemeinen Formeln (X) oder (XI) 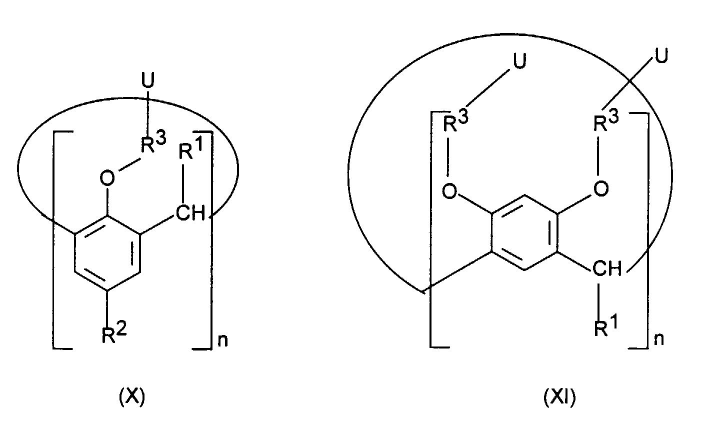 übergeführt werden,
wobei das Reagenz R Hal-CH₂-C(O)-U, U-C(O)-U, U-C(O)-C(O)-U, Ethylenoxid oder Hal-CH₂-CHR¹-Hal mit R¹ mit obiger Bedeutung und das Symbol U Halogen, OR¹ oder N(R¹)₂ mit R¹ mit obiger Definition, bedeutet.

5. Verwendung der Verbindungen der Formel (I) und (II) zum Stabilisieren von organischem Material, insbesonders als Lichtschutzmittel für Kunststoffe Anstrichmittel, Lacke und Öle.

6. Verfahren zum Stabilisieren von organischen Material gegen den zerstörerischen Abbau durch Licht und Wärme, dadurch gekennzeichnet, daß man dem zu stabilisierenden Material eine Verbindung der Formel (I) oder (II) nach den Ansprüchen 1 bis 3 in einer Konzentration von 0,001 bis 5 Gew.-%, bezogen auf das organische Material, vor, während oder nach seiner Herstellung zusetzt.

7. Verfahren gemäß Anspruch 6, dadurch gekennzeichnet daß die Zusatzmenge 0,02 bis 1 Gew.-% beträgt.

8. Stabilisiertes organisches Material, welches mindestens eine der oben genannten Verbindungen der Formel (I) oder (II) enthält.
